# EUROPEAN PATENT SPECIFICATION

(11) **EP 3 311 853 B1**
(45) Date of publication and mention of the grant of the patent: **15.07.2020**
(21) Application number: 17196748.2
(22) Date of filing: 17.10.2017
(51) Int. Cl.: A61L 26/00

(54) **PROCESS FOR PRODUCING A PACKAGED HYDROGEL DRESSING**
VERFAHREN ZUR HERSTELLUNG EINES VERPACKTEN HYDROGELVERBANDES
PROCÉDÉ DE PRODUCTION D'UN PANSEMENT HYDROGEL EMBALLÉ

(30) Priority: 20.10.2016 IT 201600105584
(43) Date of publication of application: 25.04.2018
(73) Proprietor: Universita' Degli Studi Dell'Aquila, 67100 L'Aquila (IT)
(72) Inventor: PAJEWSKI, Leonardo, 67100 L'AQUILA (IT); CORRADINI, Valentina, 67100 L'AQUILA (IT); ALECCI, Marcello, 67100 L'AQUILA (IT)
(74) Representative: Gerbino, Angelo

(56) References cited:
- WO-A1-01/30407
- WO-A1-2006/028364
- US-A- 4 871 490
- DATABASE WPI Week 201430 Thomson Scientific, London, GB; AN 2014-E96352 XP002771641, & CN 103 520 767 A (SHANDONG SAIKESAISI PHARM TECHNOLOGY CO) 22 January 2014 (2014-01-22)
- AJJI Z ET AL: "Production of hydrogel wound dressings using gamma radiation", NUCLEAR INSTRUMENTS & METHODS IN PHYSICS RESEARCH. SECTION B: BEAM INTERACTIONS WITH MATERIALS AND ATOMS, ELSEVIER BV, NL, vol. 229, no. 3-4, 1 April 2005 (2005-04-01), pages 375-380, XP027664703, ISSN: 0168-583X [retrieved on 2005-04-01]
- RITA SINGH ET AL: "Radiation synthesis of PVP/alginate hydrogel containing nanosilver as wound dressing", JOURNAL OF MATERIALS SCIENCE: MATERIALS IN MEDICINE, KLUWER ACADEMIC PUBLISHERS, BO, vol. 23, no. 11, 11 August 2012 (2012-08-11), pages 2649-2658, XP035135305, ISSN: 1573-4838, DOI: 10.1007/S10856-012-4730-3

## Description

The present invention refers to the manufacture of hydrogel-based bandages and bandages thus obtained, which are particularly applicable for use in the medical and cosmetic fields.

US Patent No. 4,871,490 issued on December 30, 1987, and having as inventors Janusz Rosiak, Anna Rucinska-Rybus and Wladyslaw Pekala, describes a production process of such a bandage from an aqueous solution of agar and polyvinylpyrrolidone which is cooled within a cavity of a mold made of a water-insoluble polymer, for example polyethylene terephthalate (PET). After cooling to room temperature, the agar dissolved in water gives rise to a thermolabile gel, while the PVP remains soluble in the aqueous matrix surrounding the agar macromolecules. The transformation of the original solution into a thermolabile gel allows it to be transported to an irradiation facility where cross-linking of the PVP and simultaneous sterilization take place. The mold, heat-sealed in polyethylene sachets, is contained in the final packaging of the bandage which is typically in the shape of a plate.

Hydrogel bandages made according to US patent No. 4,871,490 are produced and distributed in Europe under the trade names AQUAGEL™ and NEOHEAL™. Such bandages have, on the one hand, advantages over traditional bandages, particularly since they facilitate the healing of difficult injuries (decubitus wounds, ulcers, burns, etc.) with particularly rapid times, but, on the other hand, they also have drawbacks due to the fact that the plate remains firmly glued to the mold. Therefore, to be able to extract it at the time of use, it is necessary to act with extreme delicacy. Otherwise, the plate breaks, and its functionality is compromised.

WO 2006/028 364 A1 describes a process for the production of a hydrogel bandage from polymers by means of irradiation.

Ajji Z. et al., "Production of hydrogel wound dressings using gamma radiation", Nucl. Instr. and Meth. in Phys. Res. Selection B: Beam Interactions with Materials and Atoms, Elsevier BV, NL, vol. 229, no. 3-4, 1 April 2005, pages 375-380 describes a process for the production of a hydrogel bandage using a gamma ray irradiation technique.

Singh R. et al., "Radiation synthesis of PVP/alginate hydrogel containing nanosilver as wound dressing", J. Mater. Sci.:Mater. in Med., Kluwer Academic Publishers, BO, vol. 23, no. 11, 11 August 2012, pages 2649-2658 describes the synthesis by irradiation of hydrogels containing silver nano-particles.

CN 103520767 describes a hydrogel bandage containing a water-soluble polymer, a pseudo-plastic polymer, an antibacterial agent and water.

A further known technique is described in Italian patent No. 01278419 issued April 3, 1999 with Leonardo Adamo Pajewski designated as inventor.

According to such prior art, hydrogel bandages are produced from an aqueous solution of agar and sodium alginate. By cooling the casting solution in a cavity of a mold, a thermolabile agar gel is obtained with sodium alginate dissolved in water surrounding the agar macromolecules. At this point, a further cross-linking of the alginate is caused by immersing the mold containing the hydrogel in a calcium chloride bath so that a hydrogel plate is formed. The cross-linking of the calcium alginate takes place with volumetric retraction, so that the hydrogel plate separates from the walls of the mold cavity and may then be heat-sealed in a protective plastic sachet, such as polyethylene.

The hydrogel bandage thus obtained should be produced from sterile raw materials and under strictly controlled environmental conditions ("cleanroom") to be aseptic and therefore qualifiable with the CE mark, which is essential to be effectively marketed. It would in effect be impractical to sterilize the bandage by irradiation at the end of a production process carried out under less stringent conditions, as both agar and calcium alginate are degraded by radiation.

It has thus far been shown that it is impossible to carry out the process described in Italian patent No. 01278419 at reasonable cost on an industrial scale.

An object of the present invention is thus to overcome the above-described drawbacks of the technologies pertaining to the prior art.

Such object is achieved by means of a production process for a packaged bandage, comprising the successive steps of:
- preparation at a temperature between 70 and 95°C of an aqueous solution of agar, polyvinylpyrrolidone (PVP) and sodium alginate;
- casting of said solution in a cavity of a mold and cooling at room temperature (typically 25-30°C), so as to convert said solution into a thermolabile gel;
- addition of divalent metal ions (particularly calcium or barium ions) to said gel with the formation of a body that contains an alginate hydrogel of said divalent metal and agar and that separates from the walls of the mold;
- extraction of said body from the mold;
- insertion of said body within a casing;
- sealing of said casing; and
- irradiation of said sealed casing with gamma and/or beta radiation, so as to cause the cross-linking of PVP and exert a sterilization action.

The term "thermolabile gel" means a gel that is susceptible to liquefaction by heating.

The process of the invention therefore provides for a first cross-linking of the sodium alginate in the aqueous solution of the thermolabile hydrogel by divalent metal ions, in particular calcium, which involves the volumetric retraction of the hydrogel and hence its separation from the mold walls. Such process further provides for a subsequent cross-linking (due to radiation) of the PVP dissolved in the water contained in the calcium alginate hydrogel pores, which occurs under pressure due to the volumetric retraction. This involves increasing the PVP cross-linking yield that forms the hydrogel with improved mechanical properties over the hydrogel obtained by irradiating the PVP solution in the body of the thermolabile hydrogel.

Advantageously, the preparation step of the solution is carried out at a temperature between 80 and 90°C, and in the solution the agar concentration is between 0.5 and 5%, preferably 1%; the PVP - having e.g. a weight-average molecular weight of at least 300,000 Dalton - has a concentration of 5 to 40%, preferably 10%, and the sodium alginate has a concentration of between 0.1 and 3%, preferably 0.2%. All these concentrations are expressed in mass relative to the total volume of the solution.

Typically, the mold cavity is flattened, so that the hydrogel body that forms inside it is in the shape of a plate.

Advantageously, the step of adding divalent metal ions is carried out by immersion of the mold into an aqueous calcium chloride solution, or by depositing calcium chloride in powder form or as a liquid solution on the surface of the gel.

As a bandage casing, preferably a heat-sealed sachet made of plastic material, such as polyethylene, is used.

Advantageously, the radiation is absorbed in a dose of between 10 and 60 kGy, in particular equal to 25 kGy.

Another object of the present invention is a sterile bandage obtainable by carrying out a process of the type described above, wherein calcium alginate is present on the surface.

Such a bandage may for example be used for the treatment of wounds, the transdermal delivery of drugs and the application of moisturizing masks.

Advantageous properties of the bandage of the present invention are:
- ease of extraction from the related package, which allows the patient's treatment time to be shortened;
- high calcium ion content, which exert a beneficial effect on tissue regeneration;
- good deformability and resistance to breakage, which are increased with the same PVP concentration and radiation dose when cross-linking occurs under pressure in the agar/calcium alginate hydrogel pores due to volume retraction; and
- the presence of calcium alginate on the surface, which makes the bandage less vulnerable to mildew attacks.

### EXAMPLE

The raw materials used, all biocompatible, are: polyvinylpyrrolidone (PVP) of a weight-average molecular weight of 1,000,000-1,500,000 Dalton (Povidone Catalog Basf series Kollidon 90F); agar (Agar PRS-CODEX catalog Panreac number 141792); sodium alginate (alginic acid Fluka catalog number 71238); dihydrate calcium chloride (CaCl₂*2H₂O Fisher catalog number BP 510-500); and deionized water.

Polyvinylpyrrolidone, agar and sodium alginate are each dissolved in warm, deionized water by continuously agitating the solution. Subsequently, the three solutions are combined hot in proportions such as to obtain, in the final solution, concentrations of agar equal to 1% (mass/volume, m/v), of polyvinylpyrrolidone equal to 10% (m/v), and of sodium alginate equal to 0.2% (m/v). A flattened inner cavity of a mold is filled with the solution thus obtained, which is allowed to cool at room temperature. Following the coagulation of the agar, a plate-like thermolabile gel body is formed, and the mold containing it is immersed in an aqueous calcium chloride solution with a concentration equal to 0.5% (m/v). The diffusion of calcium ions within the agar gel leads to the formation of a calcium alginate and agar hydrogel with a slight volumetric retraction, facilitating the separation from the walls that delimit the cavity of the mold of the hydrogel plate. This latter is then heat-sealed within a casing made of a polyethylene sachet, which lastly is irradiated with gamma radiation absorbing a dose equal to 25 kGy thereof. This irradiation exerts a sterilizing action and causes the cross-linking of the PVP.

Thus, a hydrogel bandage is obtained in a sterile sachet to which it does not adhere. Therefore, at the time of use, the bandage may be easily extracted from the sachet once it has been opened.

## Claims

1. A process for producing a packaged bandage, comprising the successive steps of:
- preparation at a temperature comprised between 70 and 95°C of an aqueous solution of agar, polyvinylpyrrolidone (PVP) and sodium alginate;
- casting of said solution in a mold cavity and cooling at room temperature, so as to convert said solution in a thermolabile gel;
- addition of divalent metal ions to said gel with the formation of a body that contains a hydrogel of said divalent metal alginate and agar, and that separates from the walls of the mold;
- extraction of said body from the mold;
- insertion of said body within a casing;
- sealing said casing; and
- irradiation of said sealed casing with a gamma and/or beta radiation, so as to cause the cross-linking of PVP and exert sterilization action.

2. The process of claim 1, wherein in said solution the agar has a concentration of between 0.5 and 5%, the PVP has a concentration comprised between 5 and 40%, and the sodium alginate has a concentration between 0.1 and 3%, said concentrations being expressed in mass relative to the total volume of the solution.

3. The process of claim 2, wherein in said solution the agar has a concentration equal to 1%, the PVP has a concentration equal to 10%, and the sodium alginate has a concentration equal to 0.2%.

4. The process of any one of the preceding claims, wherein said PVP has a weight-average molecular weight of at least 300,000 Dalton.

5. The process of any one of the preceding claims, wherein said solution is prepared at a temperature comprised between 80 and 90°C.

6. The process of any one of the preceding claims, wherein said divalent metal ions are calcium ions.

7. The process of claim 6, wherein said addition of calcium ions is carried out by immersion of the mold into an aqueous calcium chloride solution, or by depositing calcium chloride in powder form or as a liquid solution on the surface of the gel.

8. The process of any one of the preceding claims, wherein said mold cavity is of a flat shape, so that said body is shaped like a plate.

9. The process of any one of the preceding claims, wherein said casing is a heat-sealed sachet.

10. The process of any one of the preceding claims, wherein said radiation is absorbed in a dose between 10 and 60 kGy.

11. The process according to claim 10, wherein said radiation is absorbed in a dose equal to 25 kGy.

12. A bandage obtainable by a process according to any one of the preceding claims, wherein calcium alginate is present on the surface.

13. A sterile bandage according to claim 12 having an interpenetrated structure of calcium alginate, agar and cross-linked polyvinylpyrrolidone.

## Patentansprüche

1. Verfahren zur Herstellung eines verpackten Verbandes, umfassend die aufeinander folgenden Schritte:
- Herstellung einer wässrigen Lösung von Agar, Polyvinylpyrrolidon (PVP) und Natriumalginat bei einer Temperatur zwischen 70 und 95 °C;
- Gießen der Lösung in einen Formhohlraum und Kühlen auf Raumtemperatur, um die Lösung in ein thermolabiles Gel umzuwandeln;
- Zugabe von zweiwertigen Metallionen zu dem Gel mit der Bildung eines Körpers, der ein Hydrogel aus dem zweiwertigen Metallalginat und Agar enthält, und sich von den Wänden der Form trennt;
- Herausziehen des Körpers aus der Form;
- Einsetzen des Körpers in ein Gehäuse;
- Abdichten des Gehäuses; und
- Bestrahlung des abgedichteten Gehäuses mit einer Gamma- und / oder Betastrahlung, um die Quervernetzung von PVP zu bewirken und eine Sterilisationswirkung auszuüben.

2. Verfahren nach Anspruch 1, wobei in der Lösung der Agar eine Konzentration zwischen 0,5 und 5 % aufweist, das PVP eine Konzentration zwischen 5 und 40 % aufweist und das Natriumalginat eine Konzentration zwischen 0,1 und 3 % aufweist, wobei die Konzentrationen ausgedrückt sind in der Masse bezogen auf das Gesamtvolumen der Lösung.

3. Verfahren nach Anspruch 2, wobei in der Lösung der Agar eine Konzentration von 1 %, das PVP eine Konzentration von 10 % und das Natriumalginat eine Konzentration von 0,2 % aufweist.

4. Verfahren nach einem der vorhergehenden Ansprüche, wobei das PVP ein gewichtsmittleres Molekulargewicht von mindestens 300.000 Dalton aufweist.

5. Verfahren nach einem der vorhergehenden Ansprüche, wobei die Lösung bei einer Temperatur hergestellt wird, die zwischen 80 und 90 °C liegt.

6. Verfahren nach einem der vorhergehenden Ansprüche, wobei die zweiwertigen Metallionen Calciumionen sind.

7. Verfahren nach Anspruch 6, wobei die Zugabe von Calciumionen durch Eintauchen der Form in eine wässrige Calciumchloridlösung oder durch Abscheiden von Calciumchlorid in Pulverform oder als eine flüssige Lösung auf die Oberfläche des Gels durchgeführt wird.

8. Verfahren nach einem der vorhergehenden Ansprüche, wobei der Formhohlraum eine flache Form aufweist, so dass der Körper wie eine Platte geformt ist.

9. Verfahren nach einem der vorhergehenden Ansprüche, wobei das Gehäuse ein heißversiegelter Beutel ist.

10. Verfahren nach einem der vorhergehenden Ansprüche, wobei die Strahlung in einer Dosis zwischen 10 und 60 kGy absorbiert wird.

11. Verfahren nach Anspruch 10, wobei die Strahlung in einer Dosis von 25 kGy absorbiert wird.

12. Verband, der durch ein Verfahren nach einem der vorhergehenden Ansprüche erhältlich ist, wobei Calciumalginat auf der Oberfläche vorliegt.

13. Steriler Verband nach Anspruch 12 mit einer interpenetrierten Struktur aus Calciumalginat, Agar und vernetztem Polyvinylpyrrolidon.

## Revendications

1. Procédé pour produire un bandage emballé, comprenant les étapes successives de :
- préparation à une température comprise entre 70 et 95 °C d'une solution aqueuse de gélose, de polyvinylpyrrolidone (PVP) et d'alginate de sodium ;
- coulage de ladite solution dans une cavité de moule et refroidissement à température ambiante, de manière à convertir ladite solution en un gel thermolabile ;
- ajout d'ions métalliques divalents audit gel avec la formation d'un corps qui contient un hydrogel desdits alginate de métal divalent et gélose, et qui se sépare des parois du moule ;
- extraction dudit corps du moule ;
- insertion dudit corps au sein d'un logement ;
- scellage dudit logement ; et
- irradiation dudit logement scellé avec un rayonnement gamma et/ou bêta, de manière à provoquer la réticulation de la PVP et exercer une action de stérilisation.

2. Procédé selon la revendication 1, dans lequel dans ladite solution la gélose présente une concentration entre 0,5 et 5 %, la PVP présente une concentration comprise entre 5 et 40 %, et l'alginate de sodium présente une concentration entre 0,1 et 3 %, lesdites concentrations étant exprimées en masse par rapport au volume total de la solution.

3. Procédé selon la revendication 2, dans lequel dans ladite solution la gélose présente une concentration égale à 1 %, la PVP présente une concentration égale à 10 % et l'alginate de sodium présente une concentration égale à 0,2 %.

4. Procédé selon l'une quelconque des revendications précédentes, dans lequel ladite PVP présente un poids moléculaire moyen en poids d'au moins 300 000 daltons.

5. Procédé selon l'une quelconque des revendications précédentes, dans lequel ladite solution est préparée à une température comprise entre 80 et 90 °C.

6. Procédé selon l'une quelconque des revendications précédentes, dans lequel lesdits ions métalliques divalents sont des ions calcium.

7. Procédé selon la revendication 6, dans lequel ledit ajout d'ions calcium est mené à bien par immersion du moule dans une solution aqueuse de chlorure de calcium, ou par dépôt de chlorure de calcium sous forme de poudre ou en tant que solution liquide sur la surface du gel.

8. Procédé selon l'une quelconque des revendications précédentes, dans lequel ladite cavité de moule est de forme plate, de sorte que ledit corps est façonné comme une plaque.

9. Procédé selon l'une quelconque des revendications précédentes, dans lequel ledit logement est un sachet thermoscellé.

10. Procédé selon l'une quelconque des revendications précédentes, dans lequel ledit rayonnement est absorbé en une dose entre 10 et 60 kGy.

11. Procédé selon la revendication 10, dans lequel ledit rayonnement est absorbé en une dose égale à 25 kGy.

12. Bandage susceptible d'être obtenu par un procédé selon l'une quelconque des revendications précédentes, dans lequel de l'alginate de calcium est présent sur la surface.

13. Bandage stérile selon la revendication 12 présentant une structure interpénétrée d'alginate de calcium, de gélose et de polyvinylpyrrolidone réticulée.
